(19) **Europäisches Patentamt** / **European Patent Office** / **Office européen des brevets**

(11) **EP 2 354 988 B1**

(12) **EUROPEAN PATENT SPECIFICATION**

(45) Date of publication and mention
of the grant of the patent:
**30.11.2016 Bulletin 2016/48**

(21) Application number: **09830184.9**

(22) Date of filing: **01.12.2009**

(51) Int Cl.:
*G06F 19/00* (2011.01)    *G06F 17/30* (2006.01)

(86) International application number:
**PCT/JP2009/006521**

(87) International publication number:
**WO 2010/064414 (10.06.2010 Gazette 2010/23)**

(54) **GENE CLUSTERING PROGRAM, GENE CLUSTERING METHOD, AND GENE CLUSTER ANALYZING DEVICE**

GENCLUSTERUNGSPROGRAMM, GENCLUSTERUNGSVERFAHREN UND GENCLUSTER-ANALYSEEINRICHTUNG

PROGRAMME DE GROUPEMENT DE GÈNES, PROCÉDÉ DE GROUPEMENT DE GÈNES, ET DISPOSITIF D' ANALYSE DE GROUPES DE GÈNES

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **02.12.2008  JP 2008307328**
**20.11.2009  JP 2009265433**

(43) Date of publication of application:
**10.08.2011  Bulletin 2011/32**

(73) Proprietor: **Sony Corporation**
**Tokyo 108-0075 (JP)**

(72) Inventors:
• **POLOULIAKH, Natalia**
**Tokyo 141-0022 (JP)**
• **NOCK, Richard**
**Didier 97200**
**FORT DE FRANCE MARTINIQUE (FR)**
• **NIELSEN, Frank**
**Tokyo 141-0022 (JP)**
• **KITANO, Hiroaki**
**Tokyo 141-0022 (JP)**

(74) Representative: **Müller Hoffmann & Partner Patentanwälte mbB**
**St.-Martin-Strasse 58**
**81541 München (DE)**

(56) References cited:
**US-A1- 2003 104 394    US-A1- 2005 027 460**

• **J. Z. SONG ET AL: "The Wavelet-Based Cluster Analysis for Temporal Gene Expression Data", EURASIP JOURNAL ON BIOINFORMATICS AND SYSTEMS BIOLOGY, vol. 38, no. 10, 1 January 2007 (2007-01-01), pages 1409-7, XP055185934, ISSN: 1687-4145, DOI: 10.1046/j.1365-2958.2003.03803.x**
• **NOBUHIRO TSUBOI: 'Digital Shingo Shori ni Motozuku Idenshi Clustering' TRANSACTIONS OF INFORMATION PROCESSING SOCIETY OF JAPAN vol. 41, no. SIG7, 15 November 2000, pages 49 - 56, XP008147979**
• **TAKASHI ITO: 'Kobo Genome no Taikeiteki Kino Kaiseki: Hatsugen Seigyo Network, Tanpakushitsu Kan Sogo Sayo Network' GENOME SCIENCE NO ARATANARU CHOSEN vol. 46, no. 16, 10 December 2001, pages 2407 - 2413, XP008147925**
• **KIKUYA KATO: 'Idenshi Hatsugen Profile no Data Kaiseki' PROTEIN, NUCLEIC ACID AND ENZYME vol. 48, no. 16, 14 December 2004, pages 2300 - 2309, XP008149723**
• **HIROAKI KITANO SYSTEM BIOLOGY 01 July 2001, pages 72 - 90, XP008150657**

EP 2 354 988 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

Technical Field

The present invention relates to gene clustering programs, gene clustering methods, and gene cluster analyzing devices. More specifically, the present invention relates to gene clustering programs and the like that classify each gene into a specified cluster based on the similarity of variation in gene expression level over time.

Background Art

In the field of systems biology, the elucidation of intracellular signal networks formed by genes has been attempted based on the measurement data of variations in gene expression level, gene location, and gene activity over time.

The intracellular signal network is configured with a hierarchical network architecture that varies dynamically. Recently, there has been suggested a "bow-tie signal network" as one basic network architecture composing the intracellular signal network (Non-patent Document 1 and Non-patent Document 2).

The bow-tie signal network (hereinafter simply referred to as "bow-tie network") has a network architecture that is likened to a bow-tie, and the knot of the bow-tie is imagined to be a core molecule that works as a "classifier" for regulating a cellular response to a stimulus. That is, in the bow-tie network, a wide variety of inputs in intracellular and intercellular signal transduction are gathered in the core molecule placed in the knot. Then, the intracellular concentration of the core molecule is changed depending on the input to activate a specific gene cluster positioned downstream of the signal depending on the concentration, and consequently, a specific output is expressed.

It has been reported that the bow-tie network is involved in the signal transduction between immune cells, metabolic signal transduction (Non-patent Document 1), toll-like receptor signal transduction (Non-patent Document 2), and epidermal growth factor signal transduction (Non-patent Document 3). It has been revealed that the bow-tie network is an excellent network architecture that is robust but has flexibility for evolution (Non-patent Document 4 and Non-patent Document 5).

In the bow-tie network, genes positioned downstream of a signal are clustered into a gene cluster based on a predetermined concentration of the core molecule. In order to identify the cluster to which each gene belongs based on the measurement data of the variations in gene expression level, gene location, and gene activity over time and to analyze the bow-tie network, an excellent geometrical tool is needed for elucidating the whole network architecture to forecast the relations among clusters.

Until now, there has been developed such tools based on k-means method (Non-patent Document 6), hierarchical clustering (Non-patent Document 7), and self-organizing map (Non-patent Document 8).

However, there are disadvantages in each tool that performs arithmetic processing only in one step. That is, the hierarchical clustering prepares only an inflexible dendrogram because clusters are overlaid to make the hierarchy of individual data elements. Furthermore, the hierarchical clustering clusters genes based on one-to-one similarity, and consequently the genes that are eventually classified in one cluster may have no biological association with each other.

The tool based on the self-organizing map (SOM) (for example, "GENECLUSTER") is excellent particularly in a preliminary analysis of data but requires setting of a grid size in advance for a predictive initial value of the number of clusters. The conventional k-means method also requires setting of the number of clusters in advance, and may provide a biologically meaningless result because the clustering result depends on the set number.

"GENEPattern" (Non-patent Document 9) is obtained by horizontally-integrating these conventional tools and is the most useful tool available at the present time. However, it does not have enough performance yet in order to correctly cluster each gene based on, for example, the data on gene expression level over time for elucidating the bow-tie network.

"The Wavelet-Based Cluster Analysis for Temporal Gene Expression Data" by Song et al. (EURASIP Journal on Bioinformatics and Systems Biology, 20070101 - ISSN 1687-4145; Vol: 38, Nr: 10, Page(s): 1409 - 7) discusses the use of wavelet analysis for transformation of temporal expression data for a single gene or large numbers of genes.

Non-patent Document 1: "The Edinburghhuman metabolic network reconstruction and its functional analysis", Molecular System Biology, 2007; 3: 135.
Non-patent Document 2: "A comprehensive map of the toll-like receptor signaling network", Molecular System Biology, 2006; 2: 2006. 0015.
Non-patent Document 3: "A comprehensive pathway map of epidermal growth factor receptor signaling", Molecular System Biology, 2005; 1: 2005. 0010.
Non-patent Document 4: "Bow ties, metabolism and disease", Trends in Biotechnology, 2004; 22 (9): 446-50.

Non-patent Document 5: "Biological robustness", Nature Reviews Genetics, 2004; 5 (11): 826-37.
Non-patent Document 6: "Systematic determination of genetic network architecture", Nature Genetics, 1999; 22 (3): 281-285.
Non-patent Document 7: "Cluster analysis and display of genome-wide expression patterns", Proceeding of National Academy of Sciences, 1998; 95 (25): 14863-14868.
Non-patent Document 8: "Interpreting patterns of gene expression with self-organizing maps: Methods and application to hematopoietic differentiation", Proceeding of National Academy of Sciences, 1999; 96 (6): 2907-2912.
Non-patent Document 9: "GenePattern 2.0", Nature Genetics, 2006; 38: 500-501.

Disclosure of the Invention

Problem to be solved by the Invention

Therefore, it is a major object of the present invention to provide gene clustering tools that can perform gene clustering based on the data on gene expression level over time without a priori data forecast but with high precision.

Means for solving the Problem

The above problems are solved by the subject-matter of the independent claims.

Effect of the Invention

The present invention provides gene clustering tools that can perform gene clustering based on the data on gene expression level over time without a priori data forecast but with high precision.

Brief Description of Drawings

[Fig. 1] Fig. 1 is a flowchart showing the processing steps in the gene clustering program according to the present invention.
[Fig. 2] Fig. 2 is a diagram showing an example of the data that represents variation in gene expression level over time and that is processed by the gene clustering program according to the present invention.
[Fig. 3] Figs. 3 are conceptual diagrams showing data processing by wavelet transform.
[Fig. 4] Figs. 4 are conceptual diagrams showing a method for making a histogram of the variation in gene expression level over time.
[Fig. 5] Fig. 5 is a conceptual diagram showing a change in data dimension before and after the step of calculating a feature value.
[Fig. 6] Fig. 6 is a conceptual diagram showing symmetric nearest neighbors of a gene i.
[Fig. 7] Fig. 7 is a conceptual diagram showing conversion processing from a similarity matrix M into a Boolean matrix N.
[Figs. 8] Figs. 8 are conceptual diagrams showing the Boolean matrix and the DSS matrix.
[Fig. 9] Fig. 9 is a conceptual diagram showing data processing up to when obtaining the final clustering result in the gene clustering program according to the present invention.
[Fig. 10] Fig. 10 is a block diagram showing a constitution example of the gene cluster analyzing device according to the present invention.

Best Modes for Carrying Out the Invention

A gene clustering program according to the present invention performs at least steps (1)-(4) as defined in claim 1.
Hereinafter, each step will be described step by step.

1. Calculation of Feature Value

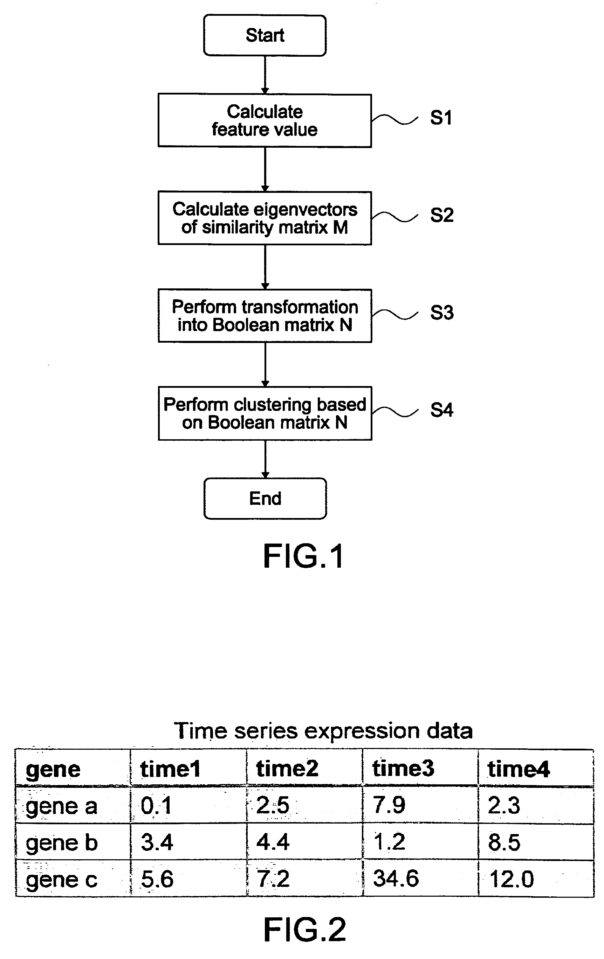

This step corresponds to the step (1) of "calculating a feature value reflecting similarity among data from the data representing variation in gene expression level over time" (see S1 in Fig. 1).
First, a feature value reflecting similarity among data is calculated from the data representing variation in gene expression level over time using the coefficients for the scaling function for D4-20 by linear regression analysis or wavelet transform (Haar wavelet transform or Daubechies wavelet transform). Fig. 2 shows an example of the data representing the variation in gene expression level over time. The shown data are expression levels measured on three genes a, b, and c at four points of time 1, 2, 3, and 4.

The linear regression analysis is a simple method for comparing variation curves representing the variation in expression level. While, the wavelet transform can collect all information over time of the variation curve. Hence, the wavelet transform can analyze even the gene that provides expression data only at one point of time and that would be excluded from the analysis in a conventional analytical method because of the incomplete measurement data.

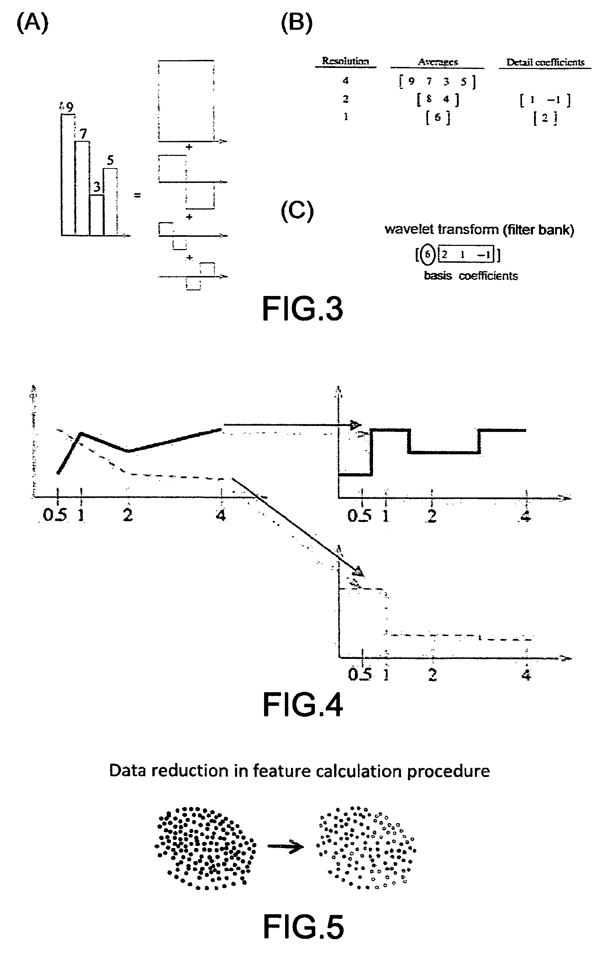

Figs. 3 show conceptual diagrams of data processing by the wavelet transform (Harr wavelet transform).

In the wavelet transform, the variation data in gene expression level over time (here, data changed from 9, 7, 3, and to 5 with time) is processed using a histogram in place of the variation curve, and the histogram is decomposed into, for example, a set of four Harr wavelet components (see Fig. 3A).

The data is represented by averages [9, 7, 3, 5] in a four-dimension, averages [8, 4] and coefficients [1, -1] in a two-dimension, and an average [6] and a coefficient [2] in a one-dimension (see Fig. 3B). Thus, the data is processed into [6 (basis), 2, 1, -1 (coefficients)] by one-dimensional wavelet transform (see Fig. 3C). The wavelet transform processes the variation data in gene expression level over time using a histogram in this manner, and therefore the most suitable fitting can be performed with a significantly smaller number of coefficients than that for the processing using a variation curve.

Figs. 4 show conceptual diagrams of a method for making a histogram from the data representing the variation in gene expression level over time as shown in Fig. 1. The variation in expression level shown as a solid line or a dotted line in Fig. 4A can be transformed into a histogram shown in Fig. 4B.

In the step, the variation data in gene expression level over time is processed as the histogram transformed in this manner and the feature value is calculated as a group of the coefficients as described above to reduce the data dimension. Fig. 5 shows a conceptual diagram of the change in data dimension before and after the step.

2. Calculation of Eigenvectors of Similarity Matrix

Next, eigenvectors of a similarity matrix M (semi-definite positive matrix M) are calculated from the calculated feature values by kernel (heat kernel) method or cosine similarity for all combinations of the genes. Hereinafter, the similarity matrix M is simply referred to as "matrix M".

This step corresponds to the step (2) of "calculating eigenvectors of a similarity matrix M from the calculated feature values for all combinations of the genes" in the gene clustering program according to the present invention (see S2 in Fig. 1).

(2-1) Matrix M by Kernel Method

When two genes are i and j (each of i and j is an integer of 1 or more), entry of i into the row and j into the column in the matrix M by the kernel method is defined as Formula (1). The entry represents the similarity between the gene i and the gene j.

[Mathematical Formula 1]

$$m_{ij} = \exp\left(-\left\|x_i - x_j\right\|_2^2 \Big/ t\right)$$

(where "$x_i$" is the gradient of a linear regression line, $\left\|\cdot\right\|_2^2$ is squared Euclidean distance, and t is any real parameter more than 0.)

(2-2) Matrix M by Cosine Similarity

The entry is defined as Formula (2) in the matrix M by the cosine similarity.

[Mathematical Formula 2]

$$m_{ij} = \frac{1}{2}\left(1 + \frac{\langle x_i, x_j \rangle}{\|x_i\|_2 \|x_j\|_2}\right)$$

3. Normalization

(3-1) Transform by FSNN

The matrix M is processed by filter by symmetric nearest neighbors (FSNN) algorithm that is similar to local linear embedding (LLE) algorithm. The processing gives a Boolean matrix (Boolean similarity matrix) that is processed more easily than the LLE algorithm. The FSNN algorithm and the LLE algorithm are described in detail in "A simple locally adaptive nearest neighbor rule with application to pollution forecasting, International Journal on Pattern Recognition and Artificial Intelligence, 17: 1-14, 2003" and "Nonlinear dimensionality reduction by locally linear embedding, Science, 290: 2323-2326, 2000", respectively.

This step corresponds to the step (3) of "transforming the similarity matrix M into a Boolean matrix N while maintaining eigenvalues of the eigenvectors" in the gene clustering program according to the present invention (see S3 in Fig. 1).

In the FSNN algorithm, q is firstly specified as an integer of 1 or more. Then, the q maximum entry is determined where the column defining the q nearest neighbors of the gene i is obtained for each row i in the matrix M.

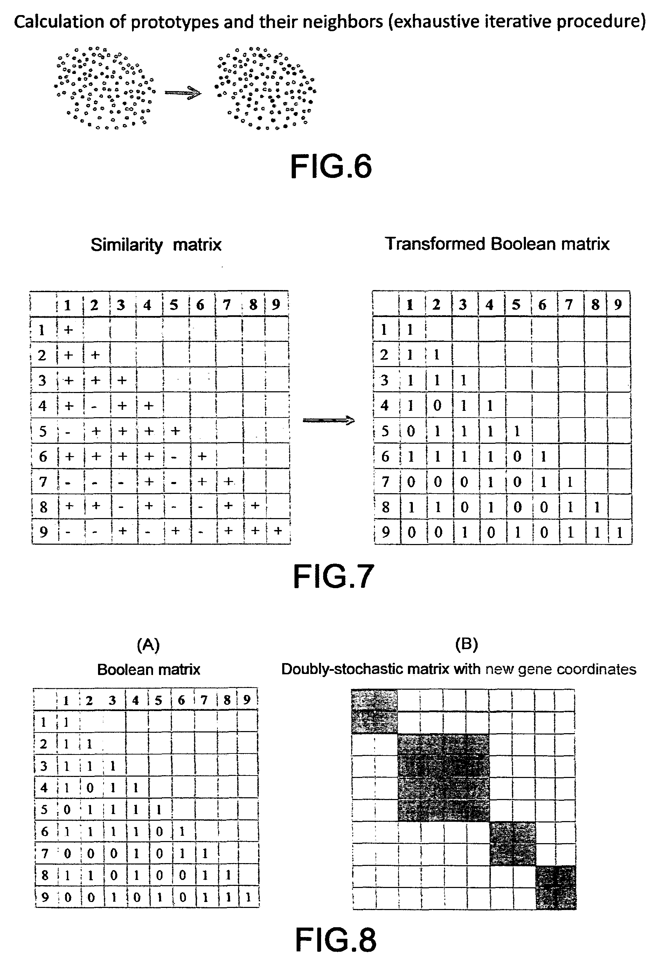

Next, the nearest neighbors of the gene i and genes to which the gene i is a nearest neighbor are collected for each row i in the matrix M to calculate the symmetric nearest neighbors of each gene i (see "A simple locally adaptive nearest neighbor rule with application to pollution forecasting, International Journal on Pattern Recognition and Artificial Intelligence, 17: 1-14, 2003"). Fig. 6 shows a conceptual diagram of the calculated symmetric nearest neighbors of the gene i.

Finally, the matrix M is replaced with a Boolean matrix N showing the symmetric nearest neighbors. Hereinafter, the Boolean matrix N is simply referred to as "matrix N". The matrix N gives 1 to mij when the genes i and j are the symmetric nearest neighbors and gives 0 when they are not the symmetric nearest neighbors. Thereby, the symmetric matrix M can be normalized into the matrix N that may be asymmetric while maintaining the eigenvalues. Fig. 7 shows a conceptual diagram of the conversion processing from the similarity matrix M into the Boolean matrix N.

The LLE algorithm reconstructs a matrix based on a standard q nearest neighbor method under a condition where neighbors of each gene are limited. That is, the LLE algorithm has a limitation that the finally obtained matrix must be symmetric. In contrast, the FSNN algorithm does not have such limitation and therefore can perform simple processing at high speed.

(3-2) Normalization of Matrix by DSS etc.

Following the normalization by FSNN algorithm, the normalization is performed by any of graph Laplacian, Markov chain, doubly-stochastic approximation (DSA) algorithm, and doubly-stochastic scaling (DSS) algorithm to reduce perturbation of the eigenvalues. Among them, the DSS algorithm is a novel algorithm developed by the inventors of the present invention for the gene clustering program according to the present invention.

In the normalization using the graph Laplacian, the matrix N is defined by Formula (3). The graph Laplacian is described in detail in "On spectral Clustering: Analysis and an Algorithm, Neural Information Processing Systems, 2001".

[Mathematical Formula 3]

$$N = D^{-1/2} M D^{-1/2}$$

(where "D" is $d_{ii} = \sum_j m_{ij}$ (diagonal matrix).)

In the normalization using the Markov chain, the matrix N is defined by Formula (4).

The Markov chain is described in detail in "Soft Membership for spectral clustering, with application to permeable language distinction, Pattern Recognition, 2008".

[Mathematical Formula 4]

$$N = D^{-1}M$$

In the normalization using the DSA algorithm, the matrix N is defined by the solution to Formula (5). The DSA algorithm is described in detail in "Doubly Stochastic Normalization for Spectral Clustering, Neural Information and Processing Systems, 2006".

[Mathematical Formula 5]

$$N = \arg\min_{X \in \Re_+^{n \times n}} \|M - X\|_F^2, s.t. X\mathbf{1} = \mathbf{1}, X = X^t$$

(where "$\|\cdot\|_F$" is Frobenius distance (the square root of the sum of the squares of the matrix elements).)
More specifically, the normalization using the DSA algorithm is performed by repeating a step of initialization where the initial value for X, X0 is set to M followed by solving Formula (6) and a step of replacing an entry Xt that does not satisfy Formula (6) with 0.

[Mathematical Formula 6]

$$X_{t+1} = X_t + \left(\frac{1}{n}I + \frac{\mathbf{1}^t X_t \mathbf{1}}{n^2} - \frac{1}{n}X_t\right)\mathbf{1}\mathbf{1}^t - \frac{1}{n}\mathbf{1}\mathbf{1}^t X_t$$

The normalization using the DSA algorithm has a disadvantage that the eigenvector has a larger deviation in the matrix N than that in the matrix M. In order to suppress the increase of the deviation in the DSA algorithm, the gene clustering program according to the present invention preferably performs the normalization by the DSS algorithm prior to the normalization using the DSA algorithm. Such normalization can maintain manifold while suppressing the deviation shift.
In the normalization using the DSS algorithm, binary search is performed based on Formula (7) to replace the matrix M with a matrix κM. The obtained matrix κM is similar to a doubly stochastic matrix and maintains the initial eigenvectors. Fig. 8B shows a conceptual diagram of the matrix κM that is obtained by the DSS algorithm and that has new gene coordinates and Fig. 8A shows a conceptual diagram of the Boolean matrix N.

[Mathematical Formula 7]

$$\kappa = \arg\min_{\gamma \in \text{int(dom}\varphi)} \left\{ \sum_i D_\varphi\left(\sum_j \gamma m_{ij} \| 1\right) + \sum_j D_\varphi\left(\sum_i \gamma m_{ij} \| 1\right) \right\}$$

More specifically, mi., m.j, and m.. are fixed to mi. = ΣjMij, m.j = Eimij, and m.. = Ei,jmij. Then, Formula (7) is varied to find the minimum value (the left parameter is at a convex of Bregman divergence) and then κ satisfying Formula (8) is determined.

[Mathematical Formula 8]

$$\sum_i m_{i.}\varphi^{[1]}(\kappa m_{i.}) + \sum_j m_{.j}\varphi^{[1]}(\kappa m_{.j}) - 2m_{..}\varphi^{[1]}(1) = 0$$

(where "$\varphi^{[1]}(x)$" is the initial derived value in divergence generator.)
Thus, κcan be represented by Formula (9), and an approximate value of κ can be obtained by simple binary search with any probability. The binary search can remarkably suppress the amount of data to be processed, and therefore can perform rapid processing.

[Mathematical Formula 9]

$$\kappa \in \left\{ \min_{i,j}\left\{ 1/m_{i.}, 1/m_{.j} \right\}, \max_{i,j}\left\{ 1/m_{i.}, 1/m_{.j} \right\} \right\}$$

The DSA algorithm described above is used for obtaining an intermediate matrix M between the initial manifold and clustering prediction described next. In order to approximate a geometric expression of the former manifold to an expression of the latter clustering prediction to a maximum extent, the number of DSA algorithm repeating times is preferably as small as possible. As described above, the DSS algorithm does not change the geometric expression. However, the normalization by the DSS algorithm prior to the normalization using the DSA algorithm can remarkably reduce the number of DSA algorithm repeating times.

4. Clustering

(4-1) Soft Clustering

Soft membership clustering can be performed using expectation maximization (EM) algorithm and complete positive factorization (CP) algorithm. The EM algorithm contributes to optimize the density of parameter and the CP algorithm contributes to minimize the amount of information loss.

This step corresponds to the step (4) of "clustering the data based on the Boolean matrix N" in the gene clustering program according to the present invention (see S4 in Fig. 1).

First, in the matrix N obtained in the normalization step, a cluster number k is fixed and the solution to Formula (10) is determined. A matrix G gives the probability of each gene being a soft membership of the cluster k.

[Mathematical Formula 10]

$$G = \arg\min_{X \in \Re_{+}^{n \times k}} \left\| N - XX^{t} \right\|_{F}^{2}$$

In the soft membership, one gene may be classified into two or more clusters. The soft membership can be replaced with a hard membership in which one gene belongs exactly to one cluster.

(4-2) Hard Clustering

A hard membership clustering uses Bregman k-means (BkM) method, hierarchical clustering (HC) algorithm, and affinity propagation (AP) algorithm. The BkM method has been developed by generalization of k-means method and can be applied to the membership of exponential family of distributions. The HC is a cumulative clustering algorithm. The AP algorithm converges data on a cluster based on the similarity matrix without previous setting of the cluster number depending on responsibility that is the probability of case node in a space selecting a central node exemplar and depending on availability that is the probability of a central node making another node belong to the group of the central node. Hereinafter, "responsibility" and "availability" may be also referred to as "responsibility message" and "availability message", respectively (see "Clustering by Passing Messages Between Data Points, Science, 315: 972-976, 2007").

Bregman-Arthur-Vassilvitskiiinitialization (BAV) algorithm that is developed by integration of important initialization techniques used in EM and BkM can perform hard clustering in a nearly optimal condition (see "k-Means++: the advantages of careful seeding, ACM-SIAM Symposium on Discrete Algorithms, 2007").

In the BAV algorithm, the center of a cluster is firstly selected in a random manner to prepare a prototype. Then, biased distribution at the center x determined by the probability p(x) is used for other center candidates. The probability p(x) is represented by Formula (11) and Formula (12). Here, the prototype means a state where each gene takes the most meaningful coordinate axes for the geometric structure.

[Mathematical Formula 11]

$$p(x) = \frac{D_\varphi\left(x\middle\|c_x\right)}{\sum_y D_\varphi\left(y\middle\|c_y\right)}$$

(where "$D_\varphi(a\|b)$" is Bergman divergence of generator $\Phi$ between genes a and b.)

[Mathematical Formula 12]

$$D_\varphi\left(x\middle\|c_x\right) = \min_{y \in C} D_\varphi\left(x\middle\|y\right)$$

Formula (12) represents the minimum divergence between one gene selected as the center C of a cluster and a gene X. The generator of a Bergman divergence is optional. In particular, selection of a squared Euclidean distance as the Bregman divergence goes back to Arthur-Vassilvitskii initialization.

Then, processing is performed by BkM in a similar manner to that by common k-means method to assign each gene to the center of a cluster, and the new center is calculated. Such processing is repeated. The calculation of the new center is performed by an algorithm of common k-means method (the arithmetic mean of cluster members is regarded as the new center), but the reassignment of each gene is performed by a more common rule. That is, the reassignment is performed where the gene X relates to the center c set as the solution to C satisfying Formula (13).

[Mathematical Formula 13]

$$c = \arg\min_{c' \in C} D_\varphi\left(x\middle\|c'\right)$$

The HC algorithm is performed using both the increment in the sum of the squares of the distances (ward linkage distance) and the shortest distance (single linkage distance).

In the AP algorithm, input of the algorithm is a similarity matrix M between genes. The similarity matrix M may include a negative number. Each entry of the row i and the column j in the matrix M is defined by Formula (14).

[Mathematical Formula 14]

$$m_{ij} = -D_\varphi\left(x_i\middle\|x_j\right)$$

Here, it should be noted that employment of Bergman dispersion in place of the similitude is not referred in "Clustering by Passing Messages Between Data Points, Science 315: 972-976, 2007" described above.

The AP algorithm defines a gene group that shows the most average profile in each cluster as a model. The AP algorithm is performed by repeating the replacement between the availability message and the responsibility message among genes. Formula (15) defines the availability message sent from the gene i to the gene j to be a model candidate.

[Mathematical Formula 15]

$$r_{ij} = m_{ij} - \max_{j' \neq j}\left\{a_{ij'} + m_{ij'}\right\}$$

The initial repeating processing sets the availability message to 0, and when the responsibility message is updated, the availability message is updated according to the following two rules.

First, for i different from j, the availability message sent from the gene j to be a model candidate to the gene i is represented by Formula (16).

[Mathematical Formula 16]

$$a_{ij} = \min\left\{0, r_{kk} + \sum_{i' \neq i, j} \max\left\{0, r_{i'j}\right\}\right\}$$

While, the self availability message of the gene j is calculated according to Formula (17).

[Mathematical Formula 17]

$$a_{jj} = \sum_{i' \neq j} \max\left\{0, r_{i'j}\right\}$$

In order to accelerate the convergence of the algorithm, each message is suppressed so that a new value will be equal to the sum of the value $\lambda$ times the previous value and the value (1-$\lambda$) times the updated value. The algorithm stops at a time when the model is not varied during m times-repeating processing.

The calculation of cluster is performed based on aij + rij of each gene pair. For the gene i, the value j maximizing the sum specifies i as a model when i = j or specifies a gene to be a model of the gene i. Each cluster corresponds to a model gene and a gene group modeling the gene.

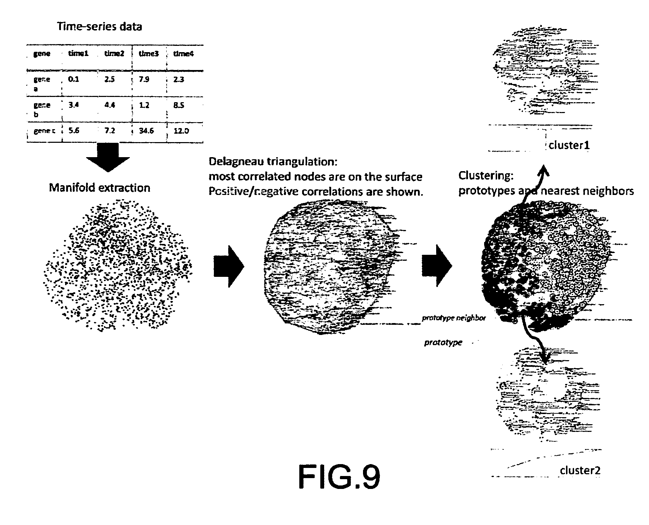

Fig. 9 is a conceptual diagram summarizing data processing up to when obtaining the final clustering result in the gene clustering program according to the present invention.

Examples of a providing medium for providing, to users, the gene clustering program that performs the various processing described above include recording media such as a magnetic disk, a CD-ROM, and a solid-state memory and communication media such as a network and a satellite.

A gene cluster analyzing device according to the present invention includes at least means (1) for calculating a feature value reflecting similarity among data from the data representing variation in gene expression level over time, means (2) for calculating eigenvectors of a similarity matrix M from the calculated feature values for all combinations of the genes, means (3) for transforming the similarity matrix M into a Boolean matrix N while maintaining eigenvalues of the eigenvectors, and means (4) for clustering the data based on the Boolean matrix N. The gene cluster analyzing device can be constituted by installing the gene clustering program onto a common computer.

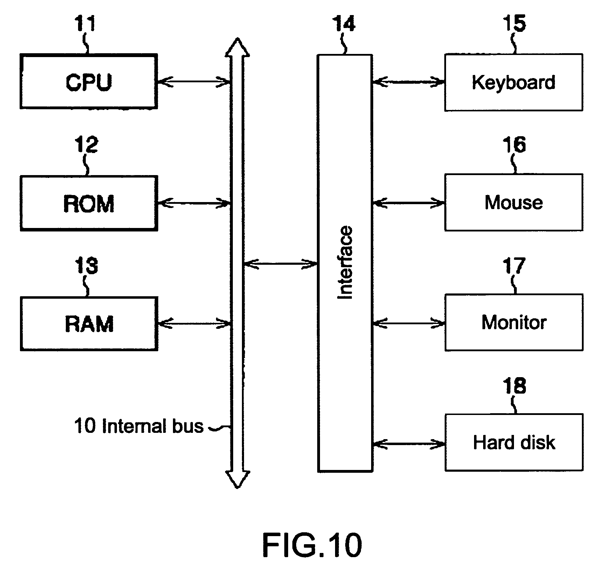

Fig. 10 is a block diagram showing a constitution example of the gene cluster analyzing device according to the present invention.

In a gene cluster analyzing device 1, an internal bus 10 includes, for example, a peripheral component interconnect (PCI) or a local bus and is interconnected to a CPU 11, a ROM 12, a RAM 13, and an interface 14. Each unit sends and receives data through the internal bus 10. The CPU 11 performs processing in accordance with the gene clustering program stored in the ROM 12. The RAM 13 stores, as needed, data, the program, and the like required by the CPU 11 for performing various processing. The interface 14 is connected to a keyboard 15 and a mouse 16, and a user uses such units for setting parameters and the like. The interface 14 outputs a control signal output from such units to the CPU 11. The interface 14 is also connected to a monitor 17 and a hard disk 18. The monitor 17 is controlled by the CPU 11 to display a predetermined image. The CPU 11 can record or read out data, a program, or the like on or from the hard disk 18 through the interface 14.

The gene clustering method according to the present invention can be performed by the gene clustering program or gene cluster analyzing device described above, and performs at least a step (1) of calculating a feature value reflecting similarity among data from the data representing variation in gene expression level over time, a step (2) of calculating eigenvectors of a similarity matrix M from the calculated feature values for all combinations of the genes, a step (3) of transforming the similarity matrix M into a Boolean matrix N while maintaining eigenvalues of the eigenvectors, and a step (4) of clustering the data based on the Boolean matrix N.

The gene clustering method can convert multidimensional data into a low-dimensional manifold by using a plurality of optimized algorithms while maintaining biological information relating to the similarity of a gene expression pattern. Therefore, it can rapidly perform advanced clustering analysis to detect a correct clustering structure without a priori data forecast.

Industrial Applicability

The gene clustering program according to the present invention can perform gene clustering based on the data on gene expression level over time without a priori data forecast but with high precision, and therefore can be effectively used for analyzing intracellular signal network such as bow-tie network.

**Claims**

1. A gene clustering program which, when executed on a computer, is adapted to cluster genes according to similarity of variation of gene expression level over time by the following steps:

   (1) calculating a feature value reflecting similarity among data from data representing variation in gene expression level over time, wherein this involves wavelet transformation of variation curves representing the variation in expression level over time;
   (2) calculating eigenvectors of a similarity matrix M from the calculated feature values for all combinations of the genes by kernel method or cosine similarity;
   (3) transforming the similarity matrix M into a Boolean matrix N while maintaining eigenvalues of the eigenvectors by filter by symmetric nearest neighbors (FSNN) algorithm; and
   (4) clustering the data based on the Boolean matrix N.

2. The gene clustering program according to claim 1, wherein, in the step (3), the matrix is normalized by any of graph Laplacian, Markov chain, doubly-stochastic approximation (DSA) algorithm, or doubly-stochastic scaling (DSS) algorithm after the transformation by the FSNN algorithm.

3. The gene clustering program according to claim 2, wherein, in the step (4), soft clustering is performed by expectation maximization (EM) algorithm and complete positive factorization (CP) algorithm.

4. The gene clustering program according to claim 3, wherein, in the step (4), hard clustering is performed by Bregman-Arthur-Vassilvitskiiinitialization (BAV) algorithm after the soft clustering.

5. A recording medium comprising the gene clustering program according to claim 1 recorded thereon readable by a computer.

6. A computer-implemented gene clustering method for clustering genes according to similarity of variation of gene expression level over time by the following steps:

   calculating a feature value reflecting similarity among data from the data representing variation in gene expression level over time, wherein this involves wavelet transformation of variation curves representing the variation in expression level over time;
   calculating eigenvectors of a similarity matrix M from the calculated feature values for all combinations of the genes by kernel method or cosine similarity;
   transforming the similarity matrix M into a Boolean matrix N while maintaining eigenvalues of the eigenvectors by filter by symmetric nearest neighbors (FSNN) algorithm; and
   clustering the data based on the Boolean matrix N.

7. A gene cluster analyzing device comprising the gene clustering program according to claim 1.

**Patentansprüche**

1. Gengruppierungsprogramm, das, wenn es auf einem Computer ausgeführt wird, dazu ausgelegt ist, Gene gemäß der Ähnlichkeit der Variation des Genexpressionsniveaus über die Zeit durch die folgenden Schritte zu gruppieren:

   (1) Berechnen eines Merkmalswerts, der die Ähnlichkeit zwischen Daten von Daten, die die Variation des Genexpressionsniveaus über die Zeit darstellen, widerspiegelt, wobei dies eine Wavelet-Transformation von Variationskurven beinhaltet, die die Variation des Expressionsniveaus über die Zeit darstellen;
   (2) Berechnen von Eigenvektoren einer Ähnlichkeitsmatrix M aus den berechneten Merkmalswerten für alle Kombinationen der Gene durch ein Kernverfahren oder eine Cosinus-Ähnlichkeit;
   (3) Transformieren der Ähnlichkeitsmatrix M in eine Boolesche Matrix N, während Eigenwerte der Eigenvektoren aufrechterhalten werden, durch einen Algorithmus zum Filtern nach den symmetrischen nächsten Nachbarn (FSNN); und
   (4) Gruppieren der Daten auf der Basis der Booleschen Matrix N.

2. Gengruppierungsprogramm nach Anspruch 1, wobei in Schritt (3) die Matrix durch irgendeinen eines Graphen-Laplace-, Markov-Ketten-, doppelt-stochastischen Näherungsalgorithmus (DSA-Algorithmus) oder doppelt-stochastischen Skalierungsalgorithmus (DSS-Algorithmus) nach der Transformation durch den FSNN-Algorithmus normiert wird.

3. Gengruppierungsprogramm nach Anspruch 2, wobei in Schritt (4) weiches Gruppieren durch einen Erwartungsmaximierungsalgorithmus (EM-Algorithmus) und vollständigen positiven Faktorisierungsalgorithmus (CP-Algorithmus) durchgeführt wird.

4. Gengruppierungsprogramm nach Anspruch 3, wobei in Schritt (4) hartes Gruppieren durch einen Bregman-Arthur-Vassilivitskii-Initialisierungsalgorithmus (BAV-Algorithmus) nach dem weichen Gruppieren durchgeführt wird.

5. Aufzeichnungsmedium mit dem darauf aufgezeichneten Gengruppierungsprogramm nach Anspruch 1, das durch einen Computer lesbar ist.

6. Computerimplementiertes Gengruppierungsverfahren zum Gruppieren von Genen gemäß der Ähnlich der Variation des Genexpressionsniveaus über die Zeit durch die folgenden Schritte:

Berechnen eines Merkmalswerts, der die Ähnlichkeit zwischen Daten von den Daten, die die Variation des Genexpressionsniveaus über die Zeit darstellen, widerspiegelt, wobei dies eine Wavelet-Transformation von Variationskurven beinhaltet, die die Variation des Expressionsniveaus über die Zeit darstellen;
Berechnen von Eigenvektoren einer Ähnlichkeitsmatrix M aus den berechneten Merkmalswerten für alle Kombinationen der Gene durch ein Kernverfahren oder eine Cosinus-Ähnlichkeit;
Transformieren der Ähnlichkeitsmatrix M in eine Boolesche Matrix N, während Eigenwerte der Eigenvektoren aufrechterhalten werden, durch einen Algorithmus zum Filtern nach den symmetrischen nächsten Nachbarn (FSNN); und
Gruppieren der Daten auf der Basis der Booleschen Matrix N.

7. Gengruppenanalysevorrichtung mit dem Gengruppierungsprogramm nach Anspruch 1.

## Revendications

1. Programme de regroupement de gènes qui, lorsqu'il est exécuté sur un ordinateur, est adapté pour regrouper des gènes en fonction d'une similarité de variation du niveau d'expression génique au cours du temps par les étapes suivantes :

(1) calcul d'une valeur de caractéristique reflétant une similarité parmi des données provenant de données représentant une variation du niveau d'expression génique au cours du temps, ceci impliquant une transformation en ondelettes de courbes de variation représentant la variation du niveau d'expression au cours du temps ;
(2) calcul de vecteurs propres d'une matrice de similarité M à partir des valeurs de caractéristiques calculées pour toutes les combinaisons des gènes par un procédé de noyau ou une similarité cosinusoïdale ;
(3) transformation de la matrice de similarité M en une matrice booléenne N cependant que les valeurs propres des vecteurs propres sont maintenues au moyen d'un filtre par un algorithme du plus proche voisin symétrique (FSNN) ; et
(4) regroupement des données sur la base de la matrice booléenne N.

2. Programme de regroupement de gènes selon la revendication 1, dans lequel, dans l'étape (3), la matrice est normalisée par l'un quelconque des algorithmes laplacien, de chaîne de Markov, d'approximation doublement stochastique (DSA), ou d'un algorithme de mise à l'échelle doublement stochastique (DSS) après la transformation par l'algorithme FSNN.

3. Programme de regroupement de gènes selon la revendication 2, dans lequel, dans l'étape (4), un regroupement flou est effectué par un algorithme de maximisation d'expectation (EM) et un algorithme de factorisation positive complète (CP).

4. Programme de regroupement de gènes selon la revendication 3, dans lequel, dans l'étape (4), un regroupement strict est effectué par un algorithme d'initialisation de Bregman-Arthur-Vassilvitskii (BAV) après le regroupement flou.

5. Support d'enregistrement comprenant le programme de regroupement de gènes selon la revendication 1 enregistré sur celui-ci, lisible par un ordinateur.

6. Procédé de regroupement de gènes exécuté sur un ordinateur, pour regrouper des gènes en fonction d'une similarité de variation du niveau d'expression génique au cours du temps par les étapes suivantes :

calcul d'une valeur de caractéristique reflétant une similarité parmi des données provenant de données représentant une variation du niveau d'expression génique au cours du temps, ceci impliquant une transformation en ondelettes de courbes de variation représentant la variation du niveau d'expression au cours du temps ;
calcul de vecteurs propres d'une matrice de similarité M à partir des valeurs de caractéristiques calculées pour toutes les combinaisons des gènes par un procédé de noyau ou une similarité cosinusoïdale ;
transformation de la matrice de similarité M en une matrice booléenne N cependant que les valeurs propres des vecteurs propres sont maintenues au moyen d'un filtre par un algorithme du plus proche voisin symétrique (FSNN) ; et
regroupement des données sur la base de la matrice booléenne N.

7. Dispositif d'analyse de regroupements de gènes comprenant le programme de regroupement de gènes selon la revendication 1.

**FIG.1**

Time series expression data

| gene | time1 | time2 | time3 | time4 |
|---|---|---|---|---|
| gene a | 0.1 | 2.5 | 7.9 | 2.3 |
| gene b | 3.4 | 4.4 | 1.2 | 8.5 |
| gene c | 5.6 | 7.2 | 34.6 | 12.0 |

**FIG.2**

(A)

(B)

| Resolution | Averages | Detail coefficients |
|---|---|---|
| 4 | [ 9  7  3  5 ] | |
| 2 | [ 8  4 ] | [ 1  −1 ] |
| 1 | [ 6 ] | [ 2 ] |

(C)

wavelet transform (filter bank)

[ (6) [ 2  1  −1 ] ]

basis coefficients

# FIG.3

# FIG.4

Data reduction in feature calculation procedure

# FIG.5

Calculation of prototypes and their neighbors (exhaustive iterative procedure)

## FIG.6

Similarity matrix

| | 1 | 2 | 3 | 4 | 5 | 6 | 7 | 8 | 9 |
|---|---|---|---|---|---|---|---|---|---|
| 1 | + | | | | | | | | |
| 2 | + | + | | | | | | | |
| 3 | + | + | + | | | | | | |
| 4 | + | - | + | + | | | | | |
| 5 | - | + | + | + | + | | | | |
| 6 | + | + | + | + | - | + | | | |
| 7 | - | - | - | + | - | + | + | | |
| 8 | + | + | - | + | - | - | + | + | |
| 9 | - | - | + | - | + | - | + | + | + |

Transformed Boolean matrix

| | 1 | 2 | 3 | 4 | 5 | 6 | 7 | 8 | 9 |
|---|---|---|---|---|---|---|---|---|---|
| 1 | 1 | | | | | | | | |
| 2 | 1 | 1 | | | | | | | |
| 3 | 1 | 1 | 1 | | | | | | |
| 4 | 1 | 0 | 1 | 1 | | | | | |
| 5 | 0 | 1 | 1 | 1 | 1 | | | | |
| 6 | 1 | 1 | 1 | 1 | 0 | 1 | | | |
| 7 | 0 | 0 | 0 | 1 | 0 | 1 | 1 | | |
| 8 | 1 | 1 | 0 | 1 | 0 | 0 | 1 | 1 | |
| 9 | 0 | 0 | 1 | 0 | 1 | 0 | 1 | 1 | 1 |

## FIG.7

(A)

Boolean matrix

| | 1 | 2 | 3 | 4 | 5 | 6 | 7 | 8 | 9 |
|---|---|---|---|---|---|---|---|---|---|
| 1 | 1 | | | | | | | | |
| 2 | 1 | 1 | | | | | | | |
| 3 | 1 | 1 | 1 | | | | | | |
| 4 | 1 | 0 | 1 | 1 | | | | | |
| 5 | 0 | 1 | 1 | 1 | 1 | | | | |
| 6 | 1 | 1 | 1 | 1 | 0 | 1 | | | |
| 7 | 0 | 0 | 0 | 1 | 0 | 1 | 1 | | |
| 8 | 1 | 1 | 0 | 1 | 0 | 0 | 1 | 1 | |
| 9 | 0 | 0 | 1 | 0 | 1 | 0 | 1 | 1 | 1 |

(B)

Doubly-stochastic matrix with new gene coordinates

## FIG.8

Time-series data

| gene | time1 | time2 | time3 | time4 |
|------|-------|-------|-------|-------|
| gene a | 0.1 | 2.5 | 7.9 | 2.3 |
| gene b | 3.4 | 4.4 | 1.2 | 8.5 |
| gene c | 5.6 | 7.2 | 34.6 | 12.0 |

Manifold extraction

Delagneau triangulation:
most correlated nodes are on the surface
Positive/negative correlations are shown.

Clustering:
prototypes and nearest neighbors

cluster1

prototype neighbor

prototype

cluster2

# FIG.9

FIG.10

EP 2 354 988 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SONG et al.** The Wavelet-Based Cluster Analysis for Temporal Gene Expression Data. *EURASIP Journal on Bioinformatics and Systems Biology,* vol. 38 (10), ISSN 1687-4145, 1409-7
- The Edinburgh human metabolic network reconstruction and its functional analysis. *Molecular System Biology,* 2007, vol. 3, 135
- A comprehensive map of the toll-like receptor signaling network. *Molecular System Biology,* 2006, vol. 2
- A comprehensive pathway map of epidermal growth factor receptor signaling. *Molecular System Biology,* 2005, vol. 1
- Bow ties, metabolism and disease. *Trends in Biotechnology,* 2004, vol. 22 (9), 446-50
- Biological robustness. *Nature Reviews Genetics,* 2004, vol. 5 (11), 826-37
- Systematic determination of genetic network architecture. *Nature Genetics,* 1999, vol. 22 (3), 281-285
- Cluster analysis and display of genome-wide expression patterns. *Proceeding of National Academy of Sciences,* 1998, vol. 95 (25), 14863-14868
- Interpreting patterns of gene expression with self-organizing maps: Methods and application to hematopoietic differentiation. *Proceeding of National Academy of Sciences,* 1999, vol. 96 (6), 2907-2912
- GenePattern 2.0. *Nature Genetics,* 2006, vol. 38, 500-501
- A simple locally adaptive nearest neighbor rule with application to pollution forecasting. *International Journal on Pattern Recognition and Artificial Intelligence,* 2003, vol. 17, 1-14
- Nonlinear dimensionality reduction by locally linear embedding. *Science,* 2000, vol. 290, 2323-2326
- Soft Membership for spectral clustering, with application to permeable language distinction. *Pattern Recognition,* 2008
- Doubly Stochastic Normalization for Spectral Clustering. *Neural Information and Processing Systems,* 2006
- Clustering by Passing Messages Between Data Points. *Science,* 2007, vol. 315, 972-976
- k-Means++: the advantages of careful seeding. *ACM-SIAM Symposium on Discrete Algorithms,* 2007